# EUROPEAN PATENT APPLICATION

(11) **EP 4 591 783 A1**
(43) Date of publication of application: **30.07.2025**
(21) Application number: 23868612.5
(22) Date of filing: 21.09.2023
(51) Int. Cl.: A61B 5/00, G02B 30/40, G02B 27/10, H04N 13/207, H04N 13/239

(54) **SCANNER, DATA PROCESSING DEVICE AND DATA PROCESSING METHOD**

(30) Priority: 21.09.2022 KR 20220119517; 13.09.2023 KR 20230121914
(71) Applicant: Medit Corp., Seoul 07207 (KR)
(72) Inventor: JO, Eun Gil, Seoul 07207 (KR); JANG, Kwangjin, Seoul 07207 (KR)
(74) Representative: Kim Kang, Jae Hee
(86) International application number: PCT/KR2023/014381
(87) International publication number: WO 2024/063560

(57) **Abstract**

A scanner may include a communicator configured to transmit or receive information to or from a data processing apparatus, a projector configured to emit pattern light generated based on pattern data, and a data obtainer configured to obtain two-dimensional image data of an object, wherein the data obtainer includes a first camera and a second camera spaced apart from each other, at least one of the first camera and camera is configured to obtain image data of the object to which the pattern light is projected, wherein the first camera is configured to obtain first image data, and the second camera is configured to obtain second image data, an optical path of the pattern light projected to the object coincides in an opposite direction with an optical path of incident light incident on the second camera, and the communicator is further configured to transmit, to the data processing apparatus, at least one of the second image data and the pattern data together with the first image data.

## Description

### Technical Field

An embodiment of the disclosure relates to a scanner, a data processing apparatus, and a data processing method, and more particularly, to an apparatus and method for processing oral images.

### Background Art

A scanner is used for the dental treatment of a patient. The scanner may obtain scan data of the surface of an object by using light reflected from the object.

A data processing apparatus, such as a personal computer (PC), connected to the scanner may generate a three-dimensional virtual model by using the scan data obtained by the scanner.

When the object is highly reflective like a metal, the scanner may fail to obtain accurate two-dimensional scan data of the object. For example, when the object is a metal structure, such as a crown, a prosthesis, or an orthodontic appliance, the reflectivity of light is high, and thus, an accurate shape is not scanned. In this case, a three-dimensional virtual model generated from inaccurate scan data may also be inaccurate.

Accordingly, a method and apparatus for generating an accurate three-dimensional virtual model even for an object with high reflectivity are required.

### Disclosure of Invention

### Solution to Problem

A scanner according to an embodiment includes a communicator configured to transmit or receive information to or from a data processing apparatus, a projector configured to emit pattern light generated based on pattern data, and a data obtainer configured to obtain two-dimensional image data of an object, wherein the data obtainer includes a first camera and a second camera spaced apart from each other, at least one of the first camera and the second camera is configured to obtain image data of the object to which the pattern light is projected, wherein the first camera is configured to obtain first image data, and the second camera is configured to obtain second image data, an optical path of the pattern light projected to the object coincides in an opposite direction with an optical path of incident light incident on the second camera, and the communicator is further configured to transmit, to the data processing apparatus, at least one of the second image data and the pattern data together with the first image data.

### Brief Description of Drawings

FIG. 1 is a diagram for explaining an oral image processing system according to an embodiment.
FIG. 2 is a diagram for explaining a method, performed by a scanner, of obtaining surface data, according to an embodiment.
FIG. 3 is a diagram illustrating an optical path of a scanner.
FIG. 4 is a diagram illustrating an optical path of a scanner, according to an embodiment.
FIG. 5 is a diagram illustrating the state in which a scanner obtains different types of data based on objects, according to an embodiment.
FIG. 6 is a diagram illustrating a state in which a scanner obtains two-dimensional image data of an object by scanning the object, according to an embodiment.
FIG. 7 is a diagram illustrating a state in which a projector and a second camera included in a scanner are coaxially arranged, according to an embodiment.
FIG. 8 is a diagram illustrating some of components of a scanner according to an embodiment.
FIG. 9 is a diagram illustrating a three-dimensional oral model of an object obtained from image data obtained using the scanner shown in FIG. 8.
FIG. 10 is a diagram illustrating transmission and reception of information between a scanner and a data processing apparatus, according to an embodiment.
FIG. 11 is a block diagram of a data processing system according to an embodiment.
FIG. 12 is a flowchart of a data processing method according to an embodiment.
FIG. 13 is a flowchart of a data processing method according to an embodiment.

### Mode for the Invention

In an embodiment, a scanner may be configured to alternately operate in a first scan mode and a second scan mode during a scan operation, and a data obtainer may be further configured to, when the scanner operates in the first scan mode, obtain two-dimensional image data at a first radiance, and when the scanner operates in the second scan mode, obtain two-dimensional image data at a second radiance, wherein the second radiance may be less than the first radiance.

In an embodiment, the scanner may further include a processor, wherein the processor may be configured to, based on a control signal from a data processing apparatus and at least one of scan modes of the scanner, control turning on and/or off of a second camera.

In an embodiment, a projector may include a near ultraviolet (NUV) light source, at least one of a first camera and the second camera may be a color camera including a color sensor or a black-and-white camera including a color filter, in response to the projector projecting NUV light to an object by using the NUV light source, the first camera and the second camera may be configured to obtain image data of the NUV light reflected from the object, and a communicator may be further configured to transmit, to the data processing apparatus, the image data of the NUV light reflected from the object.

A data processing apparatus according to an embodiment includes a communicator configured to transmit or receive information to or from a scanner including a projector, a first camera, and a second camera, and a processor configured to execute at least one instruction, wherein the processor is further configured to receive, from the scanner through the communicator, at least one of second image data obtained by the second camera and pattern data of the projector together with first image data obtained by the first camera, wherein an optical path of pattern light generated based on the pattern data and projected to the object coincides in an opposite direction with an optical path of incident light incident on the second camera, and generate a three-dimensional oral model of the object by comparing a pattern included in the first image data with a pattern corresponding to the pattern data.

In an embodiment, the processor may be further configured to generate the three-dimensional oral model of the object by obtaining depth information through finding a matching point between the pattern included in the first image data and the pattern corresponding to the pattern data.

In an embodiment, the processor may be further configured to, based on at least one of a user control signal, an identification signal indicating that the object is a metal, and the three-dimensional oral model of the object not being obtained from the first image data and the second image data, generate the three-dimensional oral model of the object by comparing the pattern included in the first image data with the pattern corresponding to the pattern data.

In an embodiment, the processor may be further configured to receive, from the scanner through the communicator, image data obtained at a first radiance when the scanner is in a first scan mode and image data obtained at a second radiance when the scanner is in a second scan mode, wherein the second radiance is less than the first radiance, and generate the three-dimensional oral model by using, as the first image data, image data obtained by the first camera among the image data obtained at the second radiance.

In an embodiment, the processor may be further configured to apply lens distortion of the second camera to the pattern data, and generate the three-dimensional oral model of the object by comparing a pattern corresponding to the pattern data to which the lens distortion of the second camera is applied with the pattern included in the first image data.

In an embodiment, at least one of the first camera and the second camera may be a color camera including a color sensor or a black-and-white camera including a color filter, the communicator may be further configured to, in response to the projector projecting near ultraviolet (NUV) light by using an NUV light source, receive, from the scanner, image data of the NUV light reflected from the object, the image data being obtained by the first camera and the second camera, and the processor may be further configured to generate, from the image data of the NUV light reflected from the object, the three-dimensional oral model in which an area in a certain wavelength band is identified.

A data processing method according to an embodiment includes emitting, by using a projector included in a scanner, pattern light generated based on pattern data, obtaining, by using a first camera included in the scanner, first image data of an object to which the pattern light is projected, and generating a three-dimensional oral model of the object by comparing a pattern included in the first image data with a pattern corresponding to the pattern data, wherein an optical path of the pattern light projected to the object coincides in an opposite direction with an optical path of incident light incident on a second camera included in the scanner.

In an embodiment, the generating of the three-dimensional oral model of the object may include generating the three-dimensional oral model of the object by obtaining depth information through finding a matching point between the pattern included in the first image data with the pattern corresponding to the pattern data.

In an embodiment, the generating of the three-dimensional oral model of the object may include, based on at least one of a user control signal, an identification signal indicating that the object is a metal, and the three-dimensional oral model of the object not being obtained from the first image data and the second image data, generating the three-dimensional oral model of the object by comparing the pattern included in the first image data with the pattern corresponding to the pattern data.

In an embodiment, the obtaining of the first image data and second image data may include, when the scanner is in a first scan mode, obtaining image data at a first radiance, and when the scanner is in a second scan mode, obtaining image data at a second radiance, wherein the second radiance is less than the first radiance, and the generating of the three-dimensional oral model of the object may include generating the three-dimensional oral model by using, as the first image data, image data obtained by the first camera among the image data obtained at the second radiance.

In an embodiment, the data processing method may further include applying lens distortion of the second camera to the pattern data, wherein the generating of the three-dimensional oral model of the object may include generating the three-dimensional oral model of the object by comparing a pattern corresponding to the pattern data to which the lens distortion of the second camera is applied with the pattern included in the first image data.

In an embodiment, one of the first camera and the second camera may be a color camera including a color sensor or a black-and-white camera including a color filter, and the data processing method may further include projecting, by the projector, near ultraviolet (NUV) light by using an NUV light source, obtaining, by using the first camera and the second camera, image data of the NUV light reflected from the object, and generating, from the image data of the NUV light reflected from the object, the three-dimensional oral model in which an area in a certain wavelength band is identified.

A recording medium according to an embodiment may be a computer-readable recording medium having recorded thereon a program that may perform, by a computer, a data processing method including emitting, by using a projector included in a scanner, pattern light generated based on pattern data, obtaining, by using a first camera included in the scanner, first image data of an object to which the pattern light is projected, and generating a three-dimensional oral model of the object by comparing a pattern included in the first image data with a pattern corresponding to the pattern data, wherein an optical path of the pattern light projected to the object coincides in an opposite direction with an optical path of incident light incident on a second camera included in the scanner.

The data processing apparatus and the data processing method according to an embodiment may obtain, by using a near ultraviolet (NUV) light source and a color camera or a black-and-white camera including a color filter, image data of the NUV light reflected from an object.

In the present specification, the principles of the present application are explained and embodiments are presented to clarify the scope of the claims and to enable those of ordinary skill in the art to carry out the present application. Embodiments of the disclosure may be implemented in various forms.

Throughout the specification, like reference numerals or characters refer to like elements. The present application does not cover all components of the embodiments, and general knowledge in the technical field to which the present application pertains or redundant content between the embodiments is omitted. Terms "...or/er", "part", and "portion" used in the specification may be implemented as software or hardware, and according to the embodiments, a plurality of "...ors/ers", "parts", and "portions" may be implemented as a single unit or element, or a single "...or/er", "part", and "portion" may include a plurality of units or elements. Hereinafter, the operating principles and embodiments of the present application are explained with reference to the accompanying drawings.

In the present specification, an image may include an image of at least one tooth, an oral cavity including the at least one tooth, or a plaster model of the oral cavity (hereinafter referred to as an "oral image").

Also, in the present specification, an image may include a two-dimensional image of an object or a three-dimensional oral image representing the object in three dimensions. The three-dimensional oral image may be generated by three-dimensionally modeling the structure of the oral cavity based on raw data and thus may be referred to as a three-dimensional oral model. Also, the three-dimensional oral model may also be referred to as a three-dimensional scan model or three-dimensional scan data.

Hereinafter, in the present specification, the oral image may be used to collectively refer to a model or image that presents the oral cavity in two or three dimensions.

Also, in the present specification, data may refer to information necessary to express an object in two or three dimensions, for example, raw data obtained using at least one camera.

In detail, the raw data is data obtained to generate an oral image and may include data (e.g., two-dimensional data) obtained by at least one image sensor included in the scanner when the object is scanned using the scanner. Raw data obtained by the scanner may be referred to as a two-dimensional image, image data, or two-dimensional image data. The raw data may refer to two-dimensional images of different points of view obtained by a plurality of cameras when the object is scanned using the scanner.

In the above description, the raw data is described as a two-dimensional image, but is not limited thereto. The raw data may also include three-dimensional image data.

In the present specification, the object refers to a subject of imaging and may include a part of a body or a model replicating the part of the body.

Light projected by a projector is reflected from the object and incident on two cameras included in the scanner. When the object has high reflectivity like a metal, the amount of light reflected from the object and incident on the two cameras may vary. In this case, the scanner may not obtain accurate two-dimensional scan data of the object, and a three-dimensional virtual model generated from inaccurate two-dimensional scan data may also be inaccurate.

The embodiment of the disclosure is to overcome the aforementioned problems and to provide a method and apparatus for generating an accurate three-dimensional virtual model of an object even when the object has high reflectivity.

Hereinafter, the embodiments are described in detail with reference to the drawings.

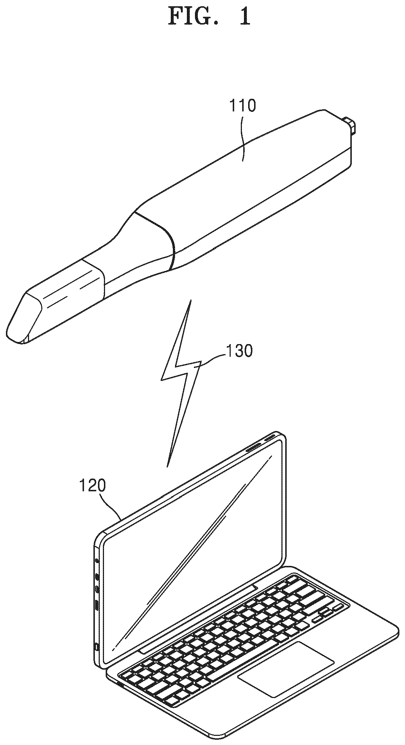

FIG. 1 is a diagram for explaining an oral image processing system according to an embodiment.

Referring to FIG. 1, an oral image processing system may include a scanner 110 and a data processing apparatus 120 coupled to the scanner 110 via a communication network 130.

The scanner 110 may be a medical apparatus that obtains an image of an object. In an embodiment, the scanner 110 may include an optical three-dimensional scanner. The optical three-dimensional scanner 110 may obtain three-dimensional surface shape information by using light reflected from the object.

The scanner 110 may obtain an image of at least one of an oral cavity, an ear, a nose, an artificial structure, or a plaster model replicating the oral cavity, the ear, the nose, or the artificial structure. Alternatively, the scanner 110 may include an oral scanner that is inserted into the oral cavity and scans teeth to obtain an oral image of the oral cavity including at least one tooth. Hereinafter, for convenience of description, a case where the scanner 110 is an oral scanner is described as an example, but the disclosure is not limited thereto.

In an embodiment, the scanner 110 may be a handheld-type scanner that is held by a user in his/her hand to scan an object while moving the scanner 110. The scanner 110 may be inserted into an ear or a nose and scan the inside of the ear or the nose in a non-contact manner. However, the disclosure is not limited thereto, and the scanner 110 may also be implemented as a table scanner.

In the present disclosure, the object may refer to an object to be scanned. The object may include a part of a body or a model replicating the part of the body. The object may include an oral cavity, a plaster model or an impression model of the oral cavity, an artificial structure insertable into the oral cavity, or a plaster model, an impression model, or a dentiform replicating the artificial structure. For example, the object may include at least one of a tooth and gingiva, a plaster model or an impression model of at least one of the tooth or the gingiva, an artificial structure insertable into the oral cavity, and/or a plaster model or an impression model of the artificial structure. In this case, the artificial structure insertable into the oral cavity may include, for example, at least one of an orthodontic appliance, an implant, a crown, an inlay, an onlay, an artificial tooth, and an orthodontic auxiliary tool inserted into the oral cavity. Also, the orthodontic appliance may include at least one of a bracket, an attachment, an orthodontic screw, a lingual orthodontic appliance, and a removable orthodontic retainer.

The scanner 110 may include a main body and a tip. The tip is a portion inserted into the oral cavity and has a detachable structure and thus may be mounted on the main body. Light emitted from the main body is directed to the object through the tip, and light received from the object is directed to the main body through the tip.

The main body may include an optical portion. The optical portion may include a light emitter that projects light and a camera that obtains an image by photographing the object.

In an embodiment, the light emitter may include a projector. The light emitter may emit pattern light to the object. In an embodiment, the pattern light may include light having a patterned shape, which is emitted by the projector to the outside of the projector.

In an embodiment, the scanner 110 may include a first camera and a second camera. In an embodiment, the first camera and the second camera may be formed to be symmetrically spaced apart from each other. The first camera and the second camera may obtain first image data and second image data having different points of view with respect to the object to which the pattern light is projected, respectively.

In an embodiment, the first camera and the second camera included in the scanner 110 may obtain surface information about the object as raw data by photographing the object to which the pattern light is emitted. In an embodiment, the first camera and the second camera may obtain the first image data and the second image data of the object, respectively.

In an embodiment, an optical path of the pattern light projected to the object may coincide in an opposite direction with an optical path of incident light to the second camera.

The scanner 110 may transmit the obtained raw data to the data processing apparatus 120 via the communication network 130.

In an embodiment, the scanner 110 may transmit, to the data processing apparatus 120 via the communication network 130, at least one of the pattern light and the second image data obtained by the second camera together with the first image data obtained by the first camera.

The data processing apparatus 120 may be connected to the scanner 110 via the communication network 130 that is wired or wireless. The data processing apparatus 120 may be any electronic apparatus that may receive raw data from the scanner 110 and generate, process, display, and/or transmit an oral image based on the received raw data. For example, the data processing apparatus 120 may be a computing apparatus, such as a smart phone, a laptop computer, a desktop computer, a personal digital assistant (PDA), or a tablet personal computer (PC). Also, the data processing apparatus 120 may be present in the form of a server (or server apparatus) for processing oral images.

In an embodiment, the data processing apparatus 120 may receive, from the scanner 110, at least one of the second image data obtained by the second camera and the pattern light projected from the projector together with the first image data obtained by the first camera.

The data processing apparatus 120 may generate a three-dimensional oral image, that is, three-dimensional scan data, based on the raw data received from the scanner 110.

In an embodiment, the data processing apparatus 120 may generate a three-dimensional oral model of the object by using one of the second image data and the pattern light together with the first image data.

Objects may include low-reflectivity objects, such as enamel included in teeth or plaster models, and high-reflectivity objects, such as inlays, onlays, crowns, prostheses, and orthodontic appliances.

Alternatively, the object may by a mixture of a high-reflectivity object and a low-reflectivity object. For example, the object may be in the form of enamel on teeth and a metal crown attached to a portion of the enamel.

In an embodiment, when the object is not a high-reflectivity object, the data processing apparatus 120 may obtain a three-dimensional oral model of the object from the first image data and the second image.

In an embodiment, when the object is a high-reflectivity object, the data processing apparatus 120 may generate a three-dimensional oral model of the object by using the first image data and the pattern light.

The data processing apparatus 120 may display the three-dimensional oral image through a display, or output or transmit the three-dimensional oral image to an external apparatus.

As another example, the scanner 110 may obtain raw data through an oral scan, generate three-dimensional information by processing the obtained raw data, and transmit the three-dimensional information to the data processing apparatus 120.

In an embodiment, the scanner 110 may obtain three-dimensional information about the object by using various methods. For example, the scanner 110 may obtain three-dimensional information about the object by using a confocal scheme. Alternatively, in an embodiment, the scanner 110 may obtain three-dimensional information about the object by using an optical triangulation technique. However, this is an embodiment. The scanner 110 may obtain three-dimensional information from the raw data by using various schemes other than the confocal scheme or the optical triangulation technique, and transmit the three-dimensional information to the data processing apparatus 120.

The data processing apparatus 120 may analyze, process, and display the received three-dimensional information, and/or transmit the same to the external apparatus.

The data processing apparatus 120 may generate a three-dimensional oral model of the object by using an image, that is, a frame, of the object received from the scanner 110.

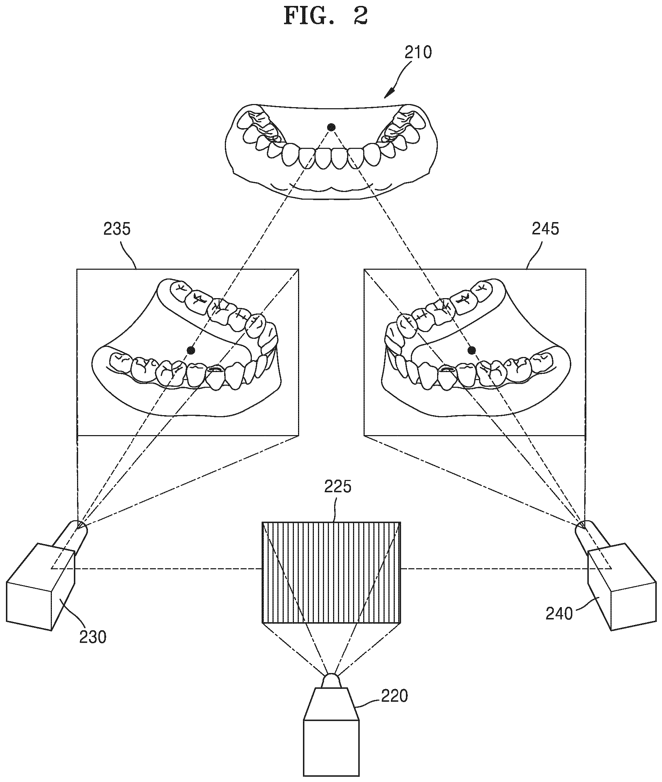

FIG. 2 is a diagram for explaining a method, performed by a scanner, of obtaining surface data, according to an embodiment.

FIG. 2 is a diagram for explaining how the scanner 110 illustrated in FIG. 1 obtains three-dimensional data by photographing an object.

In an embodiment, the scanner 110 may obtain three-dimensional data of the object by using various methods. For example, the scanner 110 may obtain three-dimensional data of the object by using the confocal scheme. The confocal scheme involves obtaining three-dimensional information about an object based on the position of a point determined using the maximum intensity of reflected light according to a reflective index of a lens that passes light emitted to the object. The scanner 110 may obtain an optical cross-sectional image with high spatial resolution by using a pinhole structure. The scanner 110 may obtain three-dimensional data by stacking two-dimensional images obtained in an axial direction.

Alternatively, in another embodiment, the scanner 110 may obtain three-dimensional information about the object by using the optical triangulation technique. The optical triangulation technique involves obtaining three-dimensional information about an object through triangulation calculations by using a triangle formed by a light source, an object to which light is emitted from the light source, and an image sensor into which light reflected from the object is input. However, this is an embodiment. The scanner 110 may obtain three-dimensional data by using various methods other than the confocal scheme or the optical triangulation technique.

Hereinafter, as an embodiment, a method by which the scanner 110 obtains three-dimensional data of the object by using the optical triangulation technique is described in more detail.

In an embodiment, the scanner 110 may obtain an image by using at least one camera and obtain three-dimensional data based on the obtained image.

In an embodiment, the scanner 110 may include an optical three-dimensional scanner. The scanner 110 may use a structured light stereo vision scheme to obtain three-dimensional data of the surface of an object 210.

The scanner 110 may include two or more cameras and a projector 220 configured to project structured light 225.

The scanner 110 may project the structured light 225 to the object 210 and obtain an L image 235 corresponding to the left field of view and an R image 245 corresponding to the right field of view respectively from an L camera 230 corresponding to the left field of view and an R camera 240 corresponding to the right field of view. The L image 235 and the R image 245 may be images of the object to which structured light (pattern light) is projected. The L image 235 and the R image 245 may be reconstructed into a three-dimensional image frame representing the surface of the object 210.

The scanner 110 may continuously obtain two-dimensional image frames including the L image 235 and the R image 245 of the object 210. The scanner 110 or the data processing apparatus 120 may obtain a three-dimensional image frame representing a surface shape of the object 210 from each two-dimensional image frame including the L image 235 and the R image 245. In FIG. 2, a case where the scanner 110 obtains three-dimensional data from two images obtained by using the two cameras 230 and 240 has been described, but this is an embodiment. The scanner 110 may also obtain an image by using only one of the two cameras 230 and 240.

The scanner 110 may obtain a plurality of two-dimensional frames by scanning an area around the object 210 at regular time intervals (e.g., 10 to 30 frames per second). The scanner 110 or the data processing apparatus 120 may obtain a plurality of three-dimensional image frames from the plurality of two-dimensional image frames.

The data processing apparatus 120 may obtain a three-dimensional oral model of the entire object 210 by merging or aligning the plurality of three-dimensional image frames.

In an embodiment, when the scanner 110 obtains an image by using only one of the two cameras 230 and 240, the data processing apparatus 120 may obtain a three-dimensional oral model of the object by using the image obtained by using only one camera and pattern light, the image being received from the scanner 110.

When the object or a portion of the object is an intraoral metal structure, such as an inlay, an onlay, a crown, a prosthesis, or an orthodontic appliance, scanning the shape is difficult when the scanner 110 scans the object by using the scheme shown in FIG. 2.

According to a three-dimensional data obtainment method of obtaining three-dimensional shape information about the surface of the object by emitting pattern light to the object, the three-dimensional shape information may be obtained only when a pattern is visible on the surface of the object. However, in the case of the metal structure, the amount of reflected light among the emitted pattern light is large, and thus, measuring the three-dimensional shape information is difficult.

Also, when the object is a metal, there may be cases where light reflected from the metal is not simultaneously incident on the L camera 230 and the R camera 240. For example, when light reflected from a point on the object is incident on only one of the L camera 230 and the R camera 240, three-dimensional data may not be obtained for the point.

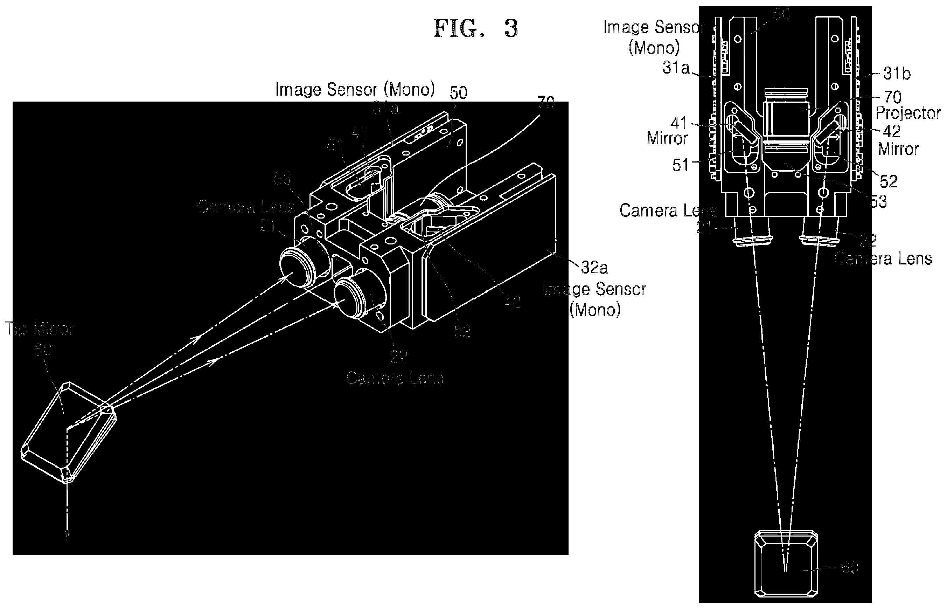

FIG. 3 is a diagram illustrating an optical path of a scanner.

Referring to FIG. 3, the scanner may include a pair of lenses 21 and 22 spaced apart from each other.

A projector 70 may be positioned at the center of the pair of lenses 21 and 22. Light emitted from the projector 70 reaches a tip by sequentially passing through an emitted light path 53 formed in a camera mounting portion 50. A path of the light is changed by a reflection mirror 60 included in the tip, and the light is projected to an object through an opening of the tip.

Light reflected from the object may sequentially enter the tip through the opening in a direction opposite to that of the light projected to the object. A path of the light incident on the tip may be changed by the reflection mirror 60, and the light may be incident on each of the pair of lenses 21 and 22. Two incident light paths 51 and 52 may be formed to transmit incident light from the pair of lenses 21 and 22 and may be formed to open to one side and the other side of the camera mounting portion 50. A path of incident light passing through the pair of lenses 21 and 22 is changed by light path changers 41 and 42, and the incident light may be emitted to image sensors 31a and 32a, which are respectively integrated on imaging boards 31b and 32b. The image sensors 31a and 32a may generate two pieces of image data by image-processing the emitted light. The image sensors 31a and 32a may be black-and-white image sensors.

When the object or a portion of the object is a metal structure, such as an inlay, an onlay, a crown, a prosthesis, or an orthodontic appliance, an accurate image is not obtained even when the object is scanned by using the scanner of the type shown in FIG. 3. This is because a metal material has high reflectivity and a large amount of light is reflected from the metal material, and thus, there are cases where patterns are not well distinguished in an image obtained by scanning a metal and light reflected from the metal surface is not simultaneously incident on the pair of lenses 21 and 22 included in a pair of cameras. That is, in the case of the metal, there are cases where light reflected from a point on the object is not simultaneously incident on both cameras, and thus, identifying a matching point in two pieces of image data obtained by the two cameras is difficult. As a result, there are challenges in measuring three-dimensional shape information.

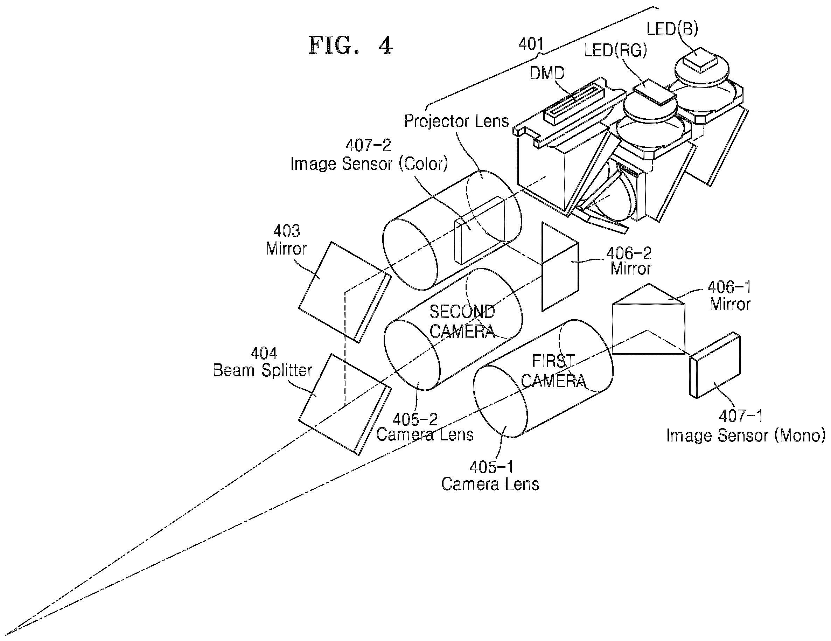

FIG. 4 is a diagram illustrating an optical path of a scanner, according to an embodiment.

FIG. 4 is a diagram illustrating some of components included in an optical portion among components of the scanner, according to an embodiment. FIG. 4 illustrates internal components of the scanner as simple figures for convenience of description, but this is only an embodiment. The internal components of the scanner may be implemented in various shapes suitable for the operation or function of each component.

Referring to FIG. 4, the scanner 110 according to an embodiment may include an optical portion. The optical portion may include a light emitter and a data obtainer that obtains an image by photographing an object.

In an embodiment, the light emitter may generate pattern light and emit light to the object.

In an embodiment, the data obtainer may include a first camera and a second camera. In an embodiment, the first camera and the second camera may be arranged side by side on a horizontal surface. In an embodiment, the first camera and the second camera may be formed to be symmetrically spaced apart from each other.

In an embodiment, the first camera and the second camera may include a first camera lens 405-1 and a second camera lens 405-2, respectively. In an embodiment, the first camera lens 405-1 and the second camera lens 405-2 may be formed to be spaced apart from each other in a direction toward a tip.

A path of light reflected from the object and incident on the tip is changed by a reflection mirror inside the tip, and the light may be incident on each of the first camera lens 405-1 and the second camera lens 405-2. A path of incident light passing through the first camera lens 405-1 and the second camera lens 405-2 may be changed by light path changers 406-1 and 406-2. The light path changers 406-1 and 406-2 may include a total reflection mirror that enables total reflection of light. However, the disclosure is not limited thereto, and the light path changers 406-1 and 406-2 may include any optical element that enables total reflection.

Light having a path changed by the light path changers 406-1 and 406-2 may be emitted to image sensors 407-1 and 407-2. The image sensors 407-1 and 407-2 may generate two pieces of image data by image-processing the emitted light.

Hereinafter, image data obtained by the image sensor 407-1 included in the first camera is referred to as first image data, and image data obtained by the image sensor 407-2 included in the second camera is referred to as second image data.

In an embodiment, both the two image sensors 407-1 and 407-2 may be monochrome image sensors. That is, in an embodiment, the first camera and the second camera may be black-and-white cameras. Image data obtained by a black-and-white camera has a higher resolution than image data obtained by a color camera that divides pixels into red, green, and blue for use. For example, the image data obtained by the black-and-white camera may have a resolution four times higher than the image data obtained by the color camera. Accordingly, the data processing apparatus 120 may identify feature points more accurately in the image data obtained by the black-and-white camera, thereby obtaining accurate scan data of the object.

In some cases, the scanner 110 may need to obtain color image of the object. Accordingly, in an embodiment, among the two image sensors 407-1 and 407-2, the image sensor 407-1 included in the first camera may be a black-and-white image sensor, and the image sensor 407-2 included in the second camera may be a color image sensor. In this case, the scanner 110 may obtain high-resolution image data by using the first camera and obtain color image data by using the second camera.

Alternatively, both the two image sensors 407-1 and 407-2 may be monochrome black-and-white image sensors, and the image sensor 407-1 included in the second camera may further include a color filter. In this case, the scanner 110 may obtain, by using the color filter, an image in which a signal in a specific wavelength band, which has passed through the color filter, among light reflected from the object is displayed.

In an embodiment, the first camera and the second camera may obtain first image data and second image data having different points of view at the same time with respect to the object to which pattern light is projected.

In an embodiment, the first camera and the second camera may be arranged such that an optical path of the first camera and an optical path of the second camera maintain a constant angle. The optical path of the first camera may refer to an optical path of incident light incident on the first camera, and the optical path of the second camera may refer to an optical path of incident light incident on the second camera.

When considering a triangle having, as vertices, a center point of each of the first camera lens 405-1 and the second camera lens 405-2 and a center point of the reflection mirror inside the tip, among internal angles of the triangle, a side connecting the center point of the first camera lens 405-1 to the center point of the reflection mirror may represent the optical path of the first camera, and a side connecting the center point of the second camera lens 405-2 to the center point of the reflection mirror may represent the optical path of the second camera. In this case, an angle between the optical path of the first camera and the optical path of the second camera is referred to as an angle (triangulation) between the two cameras.

In a stereo vision scheme, when the angle between the two cameras increases, the depth resolving power increases when a three-dimensional oral model is formed by using pieces of image data obtained by the two cameras. In contrast, when the angle between the two cameras decreases, the depth resolving power decreases. Resolving power is the ability of a lens, a screen, etc. to transmit, record, or display a fine image and may mean how detailed an object may be displayed.

Because the depth resolving power varies according to the angle between the two cameras, the angle between the two cameras needs to maintain a preset constant angle.

As described above, when the object is a metal, generating an accurate three-dimensional oral model by using two pieces of image data obtained by the two cameras is difficult.

Accordingly, the present disclosure provides a technology of generating an accurate three-dimensional oral model of an object by using one piece of image data obtained by one of the two cameras and pattern data of a projector 401.

To this end, in the scanner 110 according to an embodiment, the projector 401 may be arranged near one of the two cameras. That is, rather than the projector 70 being positioned at the center of the pair of lenses 21 and 22 as shown in FIG. 3, in the scanner 110 according to an embodiment, the projector 70 may be arranged near one of the first camera and the second camera, for example, the second camera, as shown in FIG. 4.

In an embodiment, the projector 401 may be arranged over or below the second camera. Alternatively, the projector 401 may be arranged on the side of the second camera and have a structure in which the projector 401, the second camera, and the first camera are arranged in a row in that order.

In an embodiment, the scanner 110 may project an image by using various projection schemes, for example, a cathode-ray tube (CRT) scheme, a liquid crystal display (LCD) scheme, a digital light processing (DLP) scheme, and a laser scheme.

In an embodiment, the light emitter included in the scanner 110 is a component for projecting light and may include components such as the projector 401 and a reflector.

In an embodiment, the projector 401 may include a light source, a digital micromirror device (DMD), and a projector lens.

In an embodiment, the scanner 110 may include various types of light sources. For example, the scanner 110 may include at least one light source among a lamp, a light-emitting diode (LED), and a laser.

FIG. 4 illustrates that the scanner 110 uses a red-green-blue (RGB) LED light source as a light source. In an embodiment, the scanner 110 may provide color with a combination of a red LED, a green LED, and a blue LED by using the RGB LED light source.

However, the disclosure is not limited thereto, and the scanner 110 may also use a white light source instead of the red LED, the green LED, and the blue LED as a light source.

In an embodiment, the scanner 110 may include a DMD. The scanner 110 may form a pattern by controlling each of a plurality of mirrors included in the DMD. The DMD is an assembly of microscopic mirrors, where tens of thousands of mirrors are arranged in a checkerboard pattern, and each mirror may function as a pixel. The scanner 110 may control the mirrors included in the DMD to either on or off. Each mirror has a different inclination in an on state and an off state, allowing light to either exit or not exit, thereby enabling brightness adjustment.

In an embodiment, the projector 401 of the scanner 110 may also include a spatial light modulator (SLM) other than the DMD. For example, the projector 401 may include a liquid crystal on silicon (LCoS), an LCD, a microelectromechanical system (MEMS), or the like.

In an embodiment, the projector 401 may generate pattern light having a certain pattern. In an embodiment, the pattern may be in the shape or design of pattern light that is to be generated by the projector 401 by using the DMD. In an embodiment, pattern data may be data corresponding to a pattern that is to be generated by the projector 401.

In an embodiment, the projector 401 may generate pattern light based on the pattern data and emit the pattern light to the outside of the projector 401. In an embodiment, the pattern light may have various patterns, such as a linear pattern, a diagonal pattern, and a grid pattern.

In an embodiment, the projector 401 may sequentially emit pattern light of a red LED, a green LED, and a blue LED through the projector lens provided at one end of the projector 401.

In an embodiment, emitted light emitted from the projector lens may be totally reflected by a mirror 403. In an embodiment, the emitted light that has been totally reflected by the mirror 403 and thus has a path changed may be directed to a beam splitter 404 and split into a transmitted beam and a reflected beam by the beam splitter 404. The reflected beam split by the beam splitter 404 may be emitted toward the tip and projected to the object.

As described above, according to an embodiment, an optical path of the emitted light emitted from the projector 401 may be changed by the mirror 403 and the beam splitter 404. Accordingly, although the positions of the projector 401 and the second camera are different, an optical path of projected light projected to the object may coincide in an opposite direction with the optical path of the second camera.

Also, according to an embodiment, as the projector 401 is positioned to have the same optical path as one of the two cameras, an angle between an optical path of the projector 401 and the optical path of the first camera becomes equal to an angle (triangulation) between the two cameras formed by the optical path of the first camera and the optical path of the second camera.

Accordingly, even when the three-dimensional oral model is formed by using the pattern data of the projector 401 and the first image data, the same depth resolving power as when the three-dimensional oral model is formed by using the first image data and the second image data may be maintained.

In an embodiment, the scanner 110 may transmit, to the data processing apparatus 210, at least one of the second image data obtained by the second camera and the pattern data of the projector 401 together with the first image data obtained by the first camera.

In an embodiment, the data processing apparatus 120 may generate a three-dimensional oral model based on data received from the scanner 110. In an embodiment, the data processing apparatus 120 may obtain a three-dimensional oral model of the object by using one of the second image data and the pattern data of the projector 401 together with the first image data.

In an embodiment, the data processing apparatus 120 may obtain a three-dimensional oral model of the object by using the first image data obtained by the first camera and the second image data obtained by the second camera.

In an embodiment, when the data processing apparatus 120 receives a control signal indicating that the object is a metal from a user or identifies that the object is a metal by using an artificial intelligence (AI) model, the data processing apparatus 120 may generate a three-dimensional oral model of the object by comparing a pattern included in the first image data with the pattern data of the projector 401.

Alternatively, in an embodiment, when the a three-dimensional oral model of the object is not obtained from the first image data and the second image data, the data processing apparatus 120 may generate a three-dimensional oral model of the object by comparing the first image data with the pattern data of the projector 401.

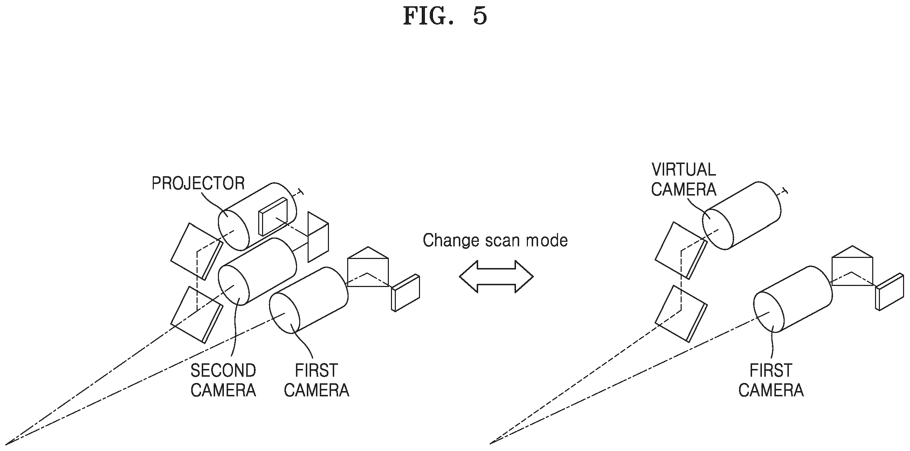

FIG. 5 is a diagram illustrating the state in which a scanner 110 obtains different types of data based on objects, according to an embodiment.

The left drawing of FIG. 5 illustrates that the scanner 110 generally obtains data by scanning an object.

In an embodiment, the scanner 110 may project pattern light to the object by using the projector 401. The scanner 110 may obtain two-dimensional image data of the object to which the pattern light is projected by using the first camera and the second camera. The scanner 110 may obtain first image data of the object by using the first camera and obtain second image data of the object by using the second camera. The first image data and the second image data may be image data obtained from different angles/points of view at the same time with respect to the object to which the pattern light is projected.

In an embodiment, the scanner 110 may transmit the first image data and the second image data to the data processing apparatus 120.

In an embodiment, the data processing apparatus may receive the first image data and the second image data from the scanner 110 and detect main features from the two pieces of image data. In an embodiment, the data processing apparatus may match feature points of the two pieces of image data to each other. In an embodiment, the data processing apparatus may calculate depth information based on matched feature point pairs. In an embodiment, the data processing apparatus may generate a depth map based on the calculated depth information. In the depth map, a depth value is assigned to each pixel position, through which three-dimensional information may be obtained.

The right drawing of FIG. 5 illustrates that the scanner 110 obtains data used to generate a three-dimensional oral model of an object when the object is a metal.

In an embodiment, the scanner 110 may project pattern light to the object. In an embodiment, the scanner 110 may obtain two-dimensional image data of a pattern formed on the object by emitting pattern light to the object by using the projector 401 and scanning the object to which the pattern light is emitted.

In an embodiment, when the object is a metal, the scanner 110 may control the second camera not to operate, as shown in the right drawing of FIG. 5. For example, in response to the object being a metal, the scanner 110 may cause the second camera not to obtain second data of the object by turning off the operation of the second camera.

In an embodiment, the scanner 110 may receive, from the data processing apparatus 120, a control signal to turn off the operation of the second camera. For example, when the data processing apparatus 120 has identified that the object is a metal, the data processing apparatus 120 may transmit, to the scanner 110, a control signal indicating that the object is a metal or transmit a control signal to turn off the operation of the second camera.

In an embodiment, in response to receiving, from the data processing apparatus 120, the control signal indicating that the object is a metal or receiving the control signal to turn off the operation of the second camera, the scanner 110 may cause the second camera not to obtain second image data by controlling the second camera not to operate.

In an embodiment, the scanner 110 may obtain first image data of the object by using the first camera, regardless of whether the object is a metal. The first image data of the object obtained by the first camera may be a patterned image in which a pattern is formed on the object. Because the pattern formed on the object is formed by the pattern light projected by the projector 401, a feature point corresponding to the pattern included in the first image data is necessarily present in the pattern data of the projector 401.

In an embodiment, the scanner 110 may transmit the first image data to the data processing apparatus 120. In an embodiment, the scanner 110 may transmit, to the data processing apparatus 120, the pattern data of the projector 401 together with the first image data.

In an embodiment, the data processing apparatus 120 may receive the first image data and the pattern data of the projector 401 from the scanner 110 and detect a feature point by using the first image data and the pattern data of the projector 401. That is, the data processing apparatus 120 may compare the pattern included in the first image data with a pattern corresponding to the pattern data of the projector 401 by using the projector 401 as a virtual camera and using the pattern data of the projector 401 instead of the second image data.

In an embodiment, the data processing apparatus 120 may detect feature points corresponding to the pattern of the first image data and the pattern corresponding to the pattern data of the projector 401 and match the detected feature points to each other. In an embodiment, the data processing apparatus may match similar feature points to each other by using information, such as a pattern, color, and texture of each feature point.

In an embodiment, the data processing apparatus may calculate depth information based on the matched feature points. One of schemes used for depth calculation involves using the distance difference (disparity) between pixels, where a greater disparity corresponds to a farther depth.

In an embodiment, the data processing apparatus may generate a depth map based on the calculated depth information. In the depth map, a depth value is assigned to each pixel position, through which three-dimensional information may be obtained.

In another embodiment, even when the object is a metal, the scanner 110 may obtain second image data of the object by using the second camera, but may not transmit the second image data to the data processing apparatus 120.

Also, in another embodiment, even when the object is a metal, the scanner 110 may obtain second image data of the object by using the second camera and transmit the second image data to the data processing apparatus 120. In this case, during generation of a three-dimensional oral model, the data processing apparatus 120 may not use the second image data in response to the object being a metal.

As described above, according to an embodiment, based on whether the object is a metal, the scanner 110 may not obtain the second image data or may not transmit the second image data to the data processing apparatus 120.

According to an embodiment, based on whether the object is a metal, the data processing apparatus 120 may generate a three-dimensional oral model of the object by using different types of data, thereby generating an accurate three-dimensional oral model according to the type of object.

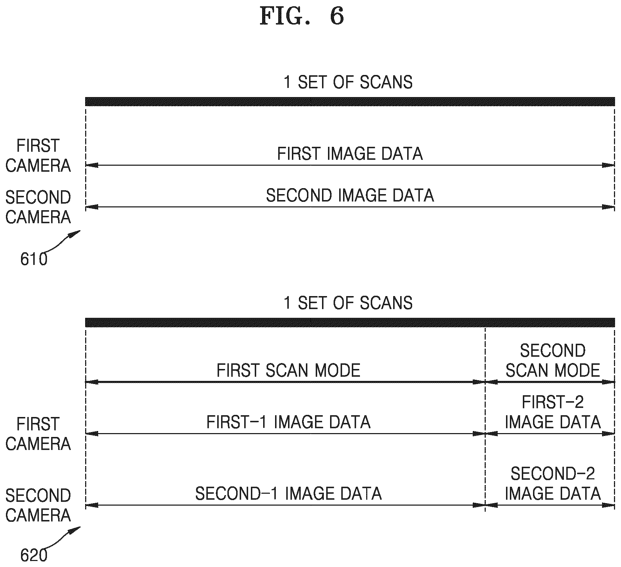

FIG. 6 is a diagram illustrating the state in which a scanner 110 obtains two-dimensional image data of an object by scanning the object, according to an embodiment.

FIG. 6 illustrates a method, performed by the scanner 110, of obtaining image data of 1 set of scans. 1 set of scans may refer to a set of image data of the same point required to generate a three-dimensional oral model.

Reference numeral 610 illustrates that, when the scanner 110 performs a scan operation, image data of an object is obtained under the same conditions, regardless of a scan mode, according to an embodiment.

In an embodiment, the scanner 110 may project pattern light to the object and obtain first image data and second image data having different points of view with respect to the object to which the pattern light is projected by using the first camera and the second camera.

For example, when 1 set of scans is 100 frames per second, the scanner 110 may obtain 100 pieces of first image data per second for the object by using the first camera, and may simultaneously obtain 100 pieces of second image data per second for the object by using the second camera.

In an embodiment, the scanner 100 may obtain image data of the object by using the method shown in reference numeral 610, regardless of the type of object, that is, regardless of whether the object is a metal. The scanner 110 may transmit all the obtained image data to the data processing apparatus 120.

The data processing apparatus 120 may generate a three-dimensional oral model of the object based on the image data of the object received from the scanner 110. In detail, the data processing apparatus 120 may select data suitable for generating a three-dimensional oral model of the object from among the image data of the object received from the scanner 110 and generate a three-dimensional oral model by combining the selected data.

For example, when the object is a metal, two-dimensional image data of the object may include a pixel that has reached a brightness/radiance threshold. The radiance threshold may refer to the state in which the radiance of a pixel is too high, preventing data from being expressed. Accordingly, when the first image data or the second image data includes a pixel having the radiance threshold, the data processing apparatus 120 is unable to generate a three-dimensional oral model of a point on the object corresponding to the pixel. Alternatively, when the object is a metal and light reflected from the metal surface is not simultaneously incident on a first camera lens and a second camera lens, there may be cases where the first image data and the second image data do not include a matching point. The data processing apparatus 120 is unable to generate a three-dimensional oral model of a point where no matching points are found between the first image data and the second image data.

In this case, the data processing apparatus 120 may generate a three-dimensional oral model of the object by using the first image data and the pattern data of the projector 401. The first image data may be a patterned image in which a pattern is formed on the object. Because the projector 401 generates pattern light based on the pattern data and projects the generated pattern light, the pattern data includes a feature point corresponding to the pattern included in the first image data. The data processing apparatus 120 may detect feature points corresponding to the pattern of the first image data and the pattern data of the projector 401, match the detected feature points, calculate depth information according to the feature points, and generate a depth map by using the depth information, thereby generating a three-dimensional oral model of the object.

Alternatively, in an embodiment, a user, such as a dentist, may scan the object, identify that the object is a metal, and transmit a control signal regarding the type of object to the data processing apparatus 120 through a user inputter or the like. In response to receiving the control signal from the user, the data processing apparatus 120 may generate a three-dimensional oral model of the object by using the first image data and the pattern data.

Alternatively, in an embodiment, the data processing apparatus 120 may identify the type of object. In an embodiment, the data processing apparatus 120 may identify the type of object from the image data received from the scanner 110 by using Al. For example, the data processing apparatus 120 may analyze a frame received from the scanner 110, identify the class of pixels included in the frame, and based on the percentage of pixels identified as a certain class among all pixels included in the frame being greater than or equal to a reference value, identify whether the class of the frame or an object included in the frame is a metal. In an embodiment, the data processing apparatus 120 may select, based on the type of object, data to be used to generate a three-dimensional oral model.

Reference numeral 620 illustrates that, when the scanner 110 performs a scan operation, the scanner 110 operates in different scan modes and obtains image data of an object, according to an embodiment.

In an embodiment, the scanner 110 may project pattern light to the object and obtain, in different modes, first image data and second image data having different points of view with respect to the object to which the pattern light is projected by using the first camera and the second camera.

In an embodiment, the scanner 110 may alternately operate in a first scan mode and a second scan mode during a scan operation. In an embodiment, the first scan mode may also be referred to as a general mode or an enamel mode. In an embodiment, the second scan mode may also be referred to as a metal mode.

In an embodiment, the first scan mode and the second scan mode may be identified according to an environment in which two-dimensional image data is obtained.

For example, when 1 set of scans is 100 frames per second, the scanner 110 may obtain 70 frames out of a total of 100 frames in the first scan mode and obtain the other 30 frames in the second scan mode.

In an embodiment, while operating in the first scan mode, the scanner 110 may obtain two-dimensional image data at a first brightness/radiance. For example, while operating in the first scan mode, the scanner 110 may obtain first-1 image data and second-1 image data of the object at the first radiance by using the first camera and the second camera, respectively.

In an embodiment, while operating in the second scan mode, the scanner 110 may obtain two-dimensional image data at a second brightness/radiance. In this case, the second radiance may be less than the first radiance. For example, while operating in the second scan mode, the scanner 110 may obtain first-2 image data and second-2 image data of the object at the second radiance by using the first camera and the second camera, respectively, the second radiance being less than the first radiance. The radiance of the two-dimensional image data obtained at the second radiance may be less than that of the two-dimensional image data obtained at the first radiance.

When the object has high reflectivity like a metal, pattern light projected to the object is more visible in a dark environment than in a bright environment. Accordingly, in an embodiment, during scanning of the object, the scanner 110 may assume that the object is a metal and perform a scan operation by adjusting the radiance of the surroundings while alternating between the first scan mode and the second scan mode, thereby obtaining image data in different environments.

In an embodiment, the scanner 110 may project light sources of different radiances in the first scan mode and the second scan mode by using the projector 401. Alternatively, in an embodiment, the first camera and the second camera may obtain image data at different radiances by adjusting the exposure time in the first scan mode and the second scan mode. The scanner 110 may obtain image data of different radiances by adjusting the light entering a lens by adjusting the time during which a camera shutter is open in the first scan mode and the second scan mode.

In an embodiment, the scanner 110 may transmit, to the data processing apparatus 120, all of the first-1 image data and the second-1 image data obtained in the first scan mode and the first-2 image data and the second-2 image data obtained in the second scan mode.

Alternatively, in an embodiment, the scanner 110 may control the second camera not to operate while operating in the second scan mode, thereby obtaining only the first-2 image data by using the first camera. In this case, the scanner 110 may transmit, to the data processing apparatus 120, only the first-1 image data and the second-1 image data obtained in the first scan mode and the first-2 image data obtained in the second scan mode.

In an embodiment, the data processing apparatus 120 may select data suitable for generating a three-dimensional oral model of the object from among data received from the scanner 110 and generate a three-dimensional oral model by combining the selected data.

For example, the data processing apparatus 120 may generally generate a three-dimensional oral model of the object by using the first-1 image data and the second-1 image data obtained in the first scan mode.

When the object is a metal and the first-1 image data and the second-1 image data obtained for the object in the first scan mode include a pixel that has reached the radiance threshold, the data processing apparatus 120 may generate, for the pixel, a three-dimensional oral model of the object by using the pattern data of the projector 401 and the first-2 image data, which is obtained by the first camera at a lower radiance, instead of generating a three-dimensional oral model of the object by using the first-1 image data and the second-1 image data.

Alternatively, in an embodiment, when a user, such as a dentist, transmits a control signal regarding the type of object to the data processing apparatus 120, in response of receiving the control signal from the user, the data processing apparatus 120 may generate a three-dimensional oral model of the object by using the first-2 image data and the pattern data of the projector 401.

Alternatively, in an embodiment, when the data processing apparatus 120 has identified the type of object on its own and the object is not a metal, the data processing apparatus 120 may generate a three-dimensional oral model of the object by using the image data obtained in the first scan mode, and when the object is a metal, generate a three-dimensional oral model of the object by comparing the first-2 image data obtained by the first camera among the image data obtained in the second scan mode with the pattern data of the projector 401.

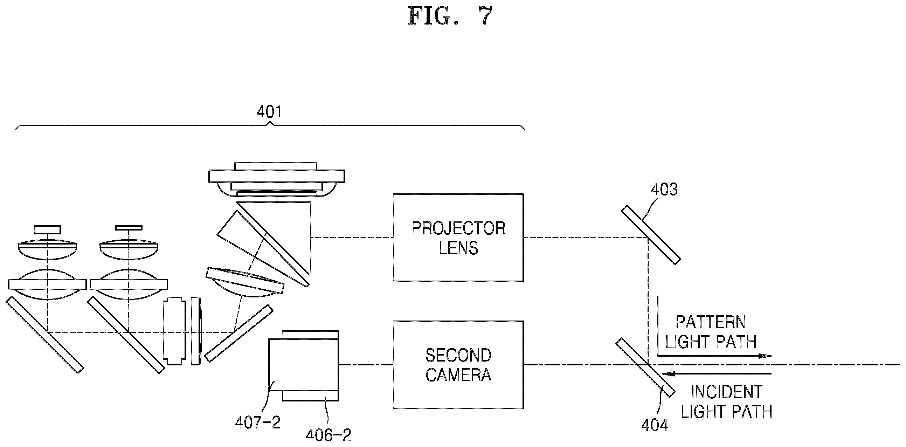

FIG. 7 is a side view of the projector 401 and the second camera included in the scanner 110, according to an embodiment.

In an embodiment, in the scanner 110, the projector 401 may be arranged near one of two cameras. For example, as shown in FIG. 7, the projector 401 may be arranged over the second camera. In an embodiment, all of components included in the projector 401 or some of the components included in the projector 401, for example, the projector lens, may be arranged over the second camera.

In an embodiment, the projector 401 may sequentially emit, through the projector lens, pattern light of a red LED, a green LED, and a blue LED generated by using the RGB LED light source and the DMD.

In an embodiment, the pattern light emitted from the projector 401 may be totally reflected by the mirror 403. In an embodiment, the emitted light that has been totally reflected by the mirror 403 and thus has a path changed may be directed to the beam splitter 404 and split into a transmitted beam and a reflected beam by the beam splitter 404. The reflected beam split by the beam splitter 404 may be emitted toward the tip and projected to the object.

In an embodiment, the pattern light projected to the object may be reflected from the object and enter the tip through the opening. The incident light incident on the tip may pass through the beam splitter 404 and be incident on the second camera. A path of the incident light incident on the second camera is changed by the light path changer 406-2, and the incident light is emitted to the image sensor 407-2 and image-processed by the image sensor 407-2 such that second image data may be generated. As described above, according to an embodiment, the pattern light from the projector 401 and the incident light incident on the second camera may have the same optical path but opposite directions. That is, the pattern light and the incident light are coaxially arranged.

According to an embodiment, as the projector 401 is positioned to have the same optical path as the second camera, an angle between an optical path of the projector 401 and an optical path of the first camera becomes equal to an angle between the two cameras formed by an optical path of the second camera and the optical path of the first camera. Accordingly, even when the three-dimensional oral model is formed based on the first camera and the pattern data of the projector 401, this case has the same depth resolving power as when the three-dimensional oral model is formed based on the image data obtained by the first camera and the second camera.

A camera lens included in a camera may have lens distortion (radial distortion) caused by the unique characteristics of the lens. Due to the radial distortion, image data of the object obtained by the camera may be expressed with a distorted shape of an actual object. For example, in the image data, the shape around the object is distorted such that a linear component may be expressed as curved as the linear component extends outward. This radial distortion increases as the angle of view increases. Also, because a wide-angle lens has the characteristic of depicting the distance between the object and the camera differently than the distance actually is, an object in front of the camera is recorded larger than the object actually is, and an object far from the camera is recorded smaller than the object actually is. Thus, the wide-angle has the characteristic of exaggerating the sense of perspective.

To correct such distortion, a process of interpreting the distortion through the difference between the image data obtained by the camera and ideal image data and correcting the interpreted distortion is required. In this case, the image data obtained by the camera and the ideal image data need to be obtained at the same time point, and when time points are different, corrected images are different. Thus, the same distortion analysis may not be applied.

In an embodiment, when the object is a metal, the data processing apparatus 120 generates a three-dimensional oral model of the object by using the first image data and the pattern data of the projector 401. In this case, the pattern data of the projector 401 corresponds to a pattern used to generate pattern light, which is different from pattern light emitted to the outside through the projector lens. As a result, the pattern data does not have radial distortion, unlike the first image data. The first image data has radial distortion caused by the first camera lens, but pattern data to be compared with the first image data does not have radial distortion, and thus, the first image data and the pattern data may not be accurately compared.

In an embodiment, because the projector 401 and the second camera are coaxially arranged to have the same optical path, distortion of the second camera may be applied to the pattern data of the projector 401.

According to an embodiment, because the pattern light from the projector 401 and the incident light incident on the second camera have the same optical path but opposite directions, the projector lens and the second camera lens have the same point of view. Accordingly, when the projector lens and the second camera lens have the same specifications, radial distortion of the second camera may be applied to the pattern data of the projector 401.

In an embodiment, the data processing apparatus 120 may interpret the radial distortion of the second camera and apply the radial distortion of the second camera to the pattern data of the projector 401. In an embodiment, the data processing apparatus 120 may identify a pixel position in the pattern data of the projector 401 that corresponds to an encrypted pattern on the object included in the second image data obtained by the second camera. In this case, the encrypted pattern may refer to assigning an ID to the pattern. In stereo vision, identifying a matching point on the object may refer to identifying a pixel position in the image data obtained by both cameras to which an object with the same ID corresponds.

The data processing apparatus 120 may compare the pattern data of the projector 401 to which the radial distortion of the second camera is applied with the pattern included in the first image data.

Therefore, according to an embodiment, by positioning the projector 401 and the second camera coaxially, the radial distortion of the second camera is applied to the pattern data of the projector 401, and the pattern data of the projector 401 to which the radial distortion is applied, is compared with the pattern of the first image data such that a more accurate three-dimensional oral model may be generated.

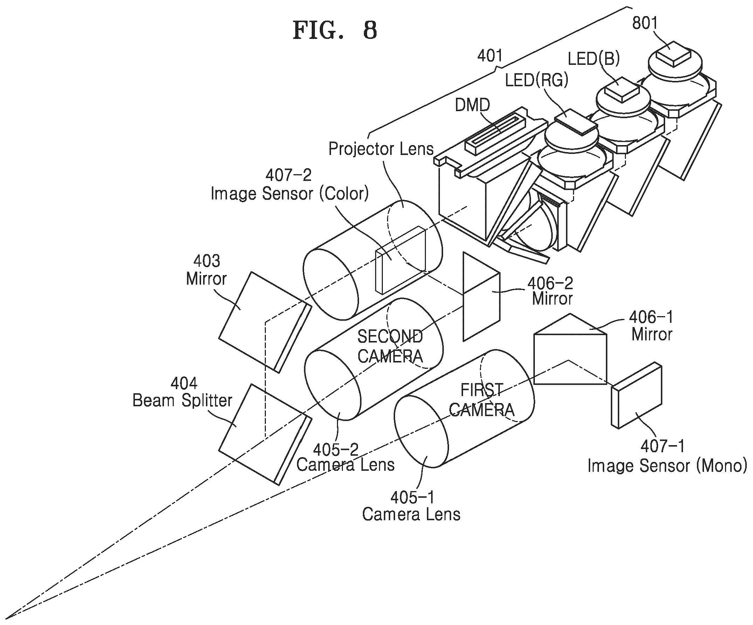

FIG. 8 is a diagram illustrating some of components of a scanner 110 according to an embodiment.

The scanner 110 shown in FIG. 8 may be an example of the scanner 110 shown in FIG. 4. Hereinafter, description redundant with those described with reference to FIG. 4 is omitted.

The scanner 110 shown in FIG. 8 may further include a near ultraviolet (NUV) light source 801, unlike the scanner 110 shown in FIG. 4.

In an embodiment, the NUV light source 801 may be a light source that emits NUV light. The NUV light is a portion of ultraviolet light that is close to visible light and refers to a portion of ultraviolet light having a wavelength close to light. For example, in the present disclosure, the NUV light emitted by the NUV light source 801 may have a wavelength of about 370 nm to about 430 nm.

In an embodiment, the scanner 110 may project NUV light to the object by using the NUV light source included in the projector 401. In an embodiment, in response to projecting NUV light to the object, the scanner 110 may receive image data of the NUV light reflected from the object, the image data being obtained by the first camera and the second camera.

Although cavity bacteria may not be identified with the naked eye, the cavity bacteria appear red to the naked eye through a fluorescence reaction when exposed to light having a specific wavelength.

In an embodiment, when NUV light having a wavelength of about 370 nm to about 430 nm is emitted to a tooth with dental caries (cavity), cavity bacteria (dental caries bacteria) fluorescently react with light, emitting light having a wavelength of about 620 nm to about 640 nm. Red is a color corresponding to a wavelength in a spectrum ranging from about 630 nm to about 700 nm.

Accordingly, when NUV light is emitted to the object and image data of the object is obtained by using a color camera including a color sensor, cavity bacteria are displayed in red in the image data.

In an embodiment, at least one of the first camera and the second camera included in the scanner 110 may be a color camera including a color sensor. The color camera may identify colors by detecting wavelengths of light emitted or radiated by the object.

For example, when the second camera is a color camera including a color sensor, image data of the object to which the NUV light is projected, obtained by the second camera, may be expressed in red according to the wavelength of light emitted by the object.

Accordingly, a user, such as a dentist, may evaluate the degree of dental caries, bacterial activity, etc. by using the image data in which cavity bacteria are displayed, thereby evaluating whether the object requires treatment.

Alternatively, in an embodiment, both the first camera and the second camera may be black-and-white cameras. As described above, because the black-and-white camera may obtain image data with a higher resolution than the color camera, when image data obtained by the black-and-white camera is used, a more accurate three-dimensional oral model may be generated.

In an embodiment, when the scanner 110 includes the NUV light source 801 and both the first camera and the second camera are black-and-white cameras, one of the first camera and the second camera may include a color filter. In an embodiment, the color filter may be a color filter that passes a red wavelength band. For example, when the second camera is a black-and-white camera including a color filter, image data obtained by the second camera may only display a portion that has fluorescently reacted with dental caries bacteria.

A mode in which the scanner 110 emits NUV light and obtains an image of the object to which the NUV light is emitted is referred to as a caries mode.

In an embodiment, the scanner 110 may operate in the caries mode separately from a scan mode in which a scan operation is performed. In an embodiment, the scanner 110 may receive, from the user and/or the data processing apparatus, a control signal to operate in the caries mode. For example, while the scanner 110 does not operate in the scan mode, the user and/or the data processing apparatus may transmit, to the scanner 110, a control signal to operate in the caries mode.

In an embodiment, when the control signal to operate in the caries mode is received, the scanner 110 may emit NUV light to the object by using the NUV light source 801 and obtain image data of the object to which the NUV light is emitted by using at least one of the first camera and the second camera. In an embodiment, the scanner 110 may obtain image data that enables identification of cavity bacteria by using the color camera including the color sensor or the black-and-white camera including the color filter among the first camera and the second camera. Alternatively, when the scanner 110 generates image data of the object to which the NUV light is emitted by using the first camera and the second camera and transmits the image data to the data processing apparatus 120, the data processing apparatus 120 may obtain image data of the object to which the NUV light is emitted by using only image data obtained by the color camera or the black-and-white camera including the color filter among the first camera and the second camera.

In an embodiment, the scanner 110 may operate in the scan mode while also operating in the caries mode. That is, the user may scan the object with the scanner 110 and simultaneously check whether the object has cavity bacteria. For example, the user and/or the data processing apparatus may transmit, to the scanner 110, a control signal to cause the scanner 110 to operate in the scan mode while also operating in the caries mode.

In an embodiment, when the scanner 110 receives the control signal to simultaneously operate in the scan mode and the caries mode, the scanner 110 may sequentially emit a red LED, a green LED, a blue LED, and NUV light by using the projector 401.

Alternatively, in an embodiment, the scanner 110 may use a white light source instead of the red LED, the green LED, and the blue LED. When the white light source is used, color information about the object may be obtained without using a separate red LED, green LED, and blue LED. In this case, the scanner 110 may use the projector 401 to sequentially emit white light and NUV light by using the white light source the NUV light source 801.

In an embodiment, the scanner 110 may operate in the first scan mode while also operating in the caries mode. In an embodiment, the scanner 110 may obtain first image data and second image data of the object to which pattern light is projected by using the first camera and the second camera. Also, the scanner 110 may obtain at least one of the first image data and the second image data of the object to which NUV light is emitted by using at least one of the first camera and the second camera. In an embodiment, the data processing apparatus 120 may generate a three-dimensional oral model of the object by using the first image data and the second image data received from the scanner 110. In this case, the three-dimensional oral model generated by the data processing apparatus 120 may be a model in which a portion that has fluorescently reacted with dental caries bacteria is displayed.

In an embodiment, the scanner 110 may operate in the second scan mode while also operating in the caries mode. In an embodiment, the data processing apparatus 120 may generate a three-dimensional oral model of the object by using pattern data and image data obtained at the second radiance when the scanner 110 is in the second scan mode. In an embodiment, the data processing apparatus 120 may generate a three-dimensional oral model of the object while generating a three-dimensional oral model of a metal on which dental caries bacteria are displayed by using at least one of the first image data and the second image data obtained for the object to which NUV light is emitted.

As described above, according to an embodiment, the scanner 110 may operate in the caries mode separately from the scan mode.

Alternatively, according to an embodiment, the scanner 110 may simultaneously operate in the scan mode and the caries mode. The data processing apparatus 120 may generate a three-dimensional oral model of the object by using the first image data and the second image data of the object to which pattern light is projected, or by using the first image data and the pattern data, while simultaneously generating a three-dimensional oral model that enables identification of cavity bacteria by using the image data obtained for the object to which NUV light is emitted.

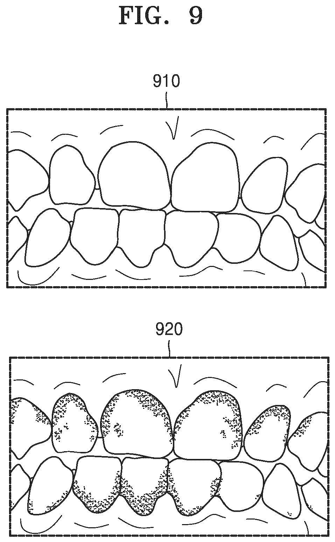

FIG. 9 is a diagram illustrating image data obtained by the scanner 110 of FIG. 8.

Referring to FIG. 9, the scanner 110 may radiate NUV light to an object by using the scanner 110 of FIG. 8. The first camera and the second camera included in the scanner 110 may obtain image data of the object to which NUV light is emitted.

Reference numeral 910 illustrates image data obtained when the object is free of dental caries bacteria, that is, cavity bacteria.

Reference numeral 920 illustrates an image obtained when the object has cavity bacteria. A portion with cavity bacteria is exposed to and fluorescently reacts with NUV light and radiates light in a certain wavelength band, and is thus expressed differently from a portion without cavity bacteria, as shown in reference numeral 920.

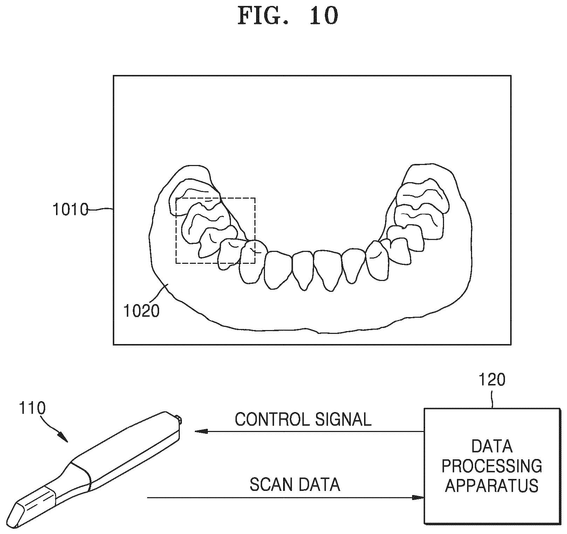

FIG. 10 is a diagram illustrating transmission and reception of information between a scanner 110 and a data processing apparatus 120, according to an embodiment.

In an embodiment, the data processing apparatus 120 may transmit a control signal to the scanner 110. The data processing apparatus 120 may control the scanner 110 by transmitting, to the scanner 110, a control signal to control the operation of the projector 401 or the data obtainer included in the scanner 110.

In an embodiment, when an object is a metal, the data processing apparatus 120 may transmit, to the scanner 110, a control signal to turn off the second camera.

In an embodiment, the data processing apparatus 120 may transmit, to the scanner 110, a control signal to operate in the scan mode or a control signal to operate in the caries mode. Alternatively, in an embodiment, the data processing apparatus 120 may transmit, to the scanner 110, a control signal to operate in the scan mode while also performing the caries mode.

The scanner 110 may transmit scan data to the data processing apparatus 120. In an embodiment, the scan data may include at least one of second image data and pattern light projected by the projector 401 together with first image data of the object.

In an embodiment, the data processing apparatus 120 may receive, from the scanner 110, at least one of pattern data of the projector 401 and the second image data together with the first image data, and generate a three-dimensional oral model of the object by using the scan data received from the scanner 110.

In an embodiment, the data processing apparatus 120 may generate a three-dimensional oral model of the object from the first image data and the second image data.

In an embodiment, when a user control signal is received, the object is identified as a metal, or a three-dimensional oral model of the object may not be obtained from the first image data and the second image data, the data processing apparatus 120 may generate a three-dimensional oral model of the object by comparing a pattern included in the first image data with the pattern data of the projector 401.

In an embodiment, the data processing apparatus 120 may generate a three-dimensional oral model 1020 by using the image data or the pattern data received from the scanner 110 and output the same through a display 1010.

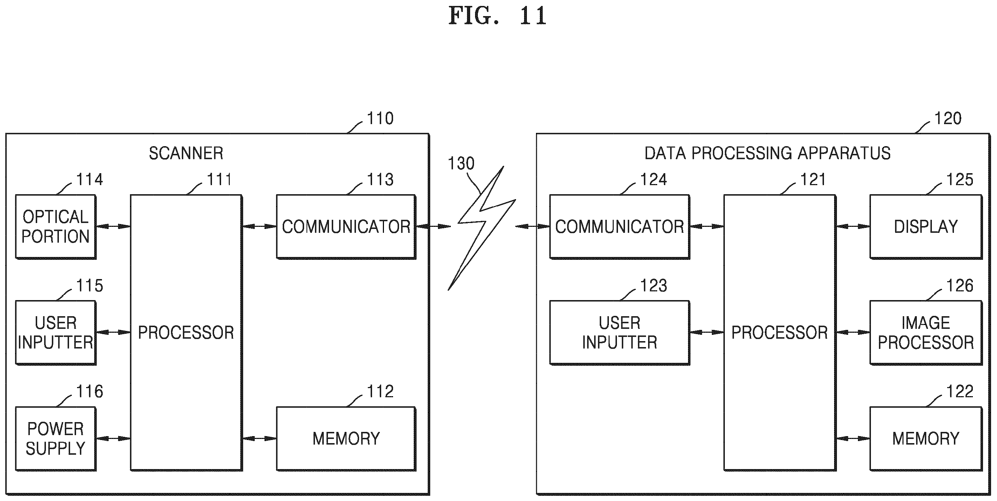

FIG. 11 is a block diagram of a data processing system according to an embodiment.

In an embodiment, the data processing system may include a scanner 110, a data processing apparatus 120, and a communication network 130. The scanner 110 of FIG. 11 may be an example of the scanner 110 that has been described with reference to FIGS. 1, 2, and 4 to 10.

The scanner 110 may obtain raw data by scanning an object. The object may include, for example, a patient's oral cavity or a tooth cast model, but is not limited thereto. The scanner 110 may generate a three-dimensional virtual model by transmitting the obtained raw data to the data processing apparatus 120 via the communication network 130 or processing the raw data, and transmit the three-dimensional virtual model to the data processing apparatus 120.

The scanner 110 may include a processor 111, a memory 112, a communicator 113, an optical portion 114, a user inputter 115, and a power supply 116.

The memory 112 may store at least one instruction. Also, the memory 112 may store at least one instruction executed by the processor 111. Also, the memory 112 may store at least one program executed by the processor 111.

The communicator 113 may communicate with the data processing apparatus 120 via a wired or wireless communication network.

In an embodiment, the communicator 113 may transmit or receive a control signal to or from the data processing apparatus 120. Also, the communicator 113 may transmit, to the data processing apparatus 120, information regarding the operating state of the scanner 110. In addition, the communicator 113 may transmit, to the data processing apparatus 120, raw data obtained by the optical portion 114.

The communicator 113 may include at least one short-range communication module that performs communication according to communication standards, such as Bluetooth, wireless fidelity (Wi-Fi), Bluetooth low energy (BLE), near-field communication (NFC)/radio frequency identification (RFID), Wi-Fi direct, ultra-wideband (UWB), or ZigBee, a long-range communication module that communicates with a server for supporting long-range communication according to long-range communication standards, and at least one port for connection to an external electronic apparatus with a wired cable for wired communication.

The optical portion 114 may include a light emitter that emits light and at least one camera that receives light reflected from the object. The light emitter may include a projector 401 that projects light from a light source.

The optical portion 114 may project pattern light or structured light by using the projector 401. The optical portion 114 may form a pattern by emitting light with a light source and controlling each of micro mirrors included in the DMD. The optical portion 114 may emit light by controlling the mirrors included in the DMD to be turned on or off. The optical portion 114 may obtain three-dimensional data representing the shape of the object by emitting pattern light to the object and scanning the object to which the pattern light is emitted.

In an embodiment, the optical portion 114 may include a first camera and a second camera symmetrically spaced apart from each other. The first camera and the second camera may obtain first image data and second image data having different points of view with respect to the object to which the pattern light is projected, respectively.

In an embodiment, the projector 401 may be formed over, below, or on one side of the second camera such that an optical path of the pattern light projected to the object coincides in an opposite direction with an optical path of incident light incident on the second camera.

In an embodiment, the optical portion 114 may include an NUV light source and project NUV light to the object by using the NUV light source.

In an embodiment, at least one of the first camera and the second camera may be a color camera including a color sensor or a black-and-white camera including a color filter.

In an embodiment, in response to the projector 401 projecting the NUV light to the object by using the NUV light source, the first camera and the second camera may obtain image data of the NUV light reflected from the object.

In this case, the communicator 113 may transmit, to the data processing apparatus 120, first image data and second image data of the NUV light reflected from the object.

In an embodiment, the scanner 110 may alternately operate in the first scan mode and the second scan mode. When the scanner 110 operates in the first scan mode, the first camera and the second camera may obtain two-dimensional image data at the first radiance. Also, when the scanner 110 operates in the second scan mode, the first camera and the second camera may obtain two-dimensional image data at the second radiance.

In an embodiment, the projector 401 may project the light source at different radiances in the first scan mode and the second scan mode. Alternatively, in an embodiment, the first camera and the second camera may obtain image data having different radiances by adjusting the exposure time in the first scan mode and the second scan mode.

The user inputter 115 may receive a user input for controlling the scanner 110. The user inputter 115 may also be referred to as a user interface.

The user inputter 115 may include a touch panel that detects a user's touch, a button that receives a user's push operation, a speech recognition apparatus including a microphone, or the like. Alternatively, the user inputter 115 may further include at least one of a wheel or a dome switch that receives a user's rotation operation and a sensor (not shown) that may recognize motions.

In an embodiment, the user may input a control signal to control the scanner to operate in the scan mode or to operate in the caries mode by using the user inputter 115.

In an embodiment, when the object is a metal, the user may input a control signal to cause the second camera not to operate by using the user inputter 115.

The power supply 116 may receive power and supply the power to each component of the scanner 110.

The processor 111 may control all the components of the scanner 110. The processor 111 may control at least one component included in the scanner 110 so that an intended operation is performed. Accordingly, even when a case where the processor 111 performs certain operations is described as an example, this may mean that the processor 111 controls at least one component included in the scanner 110 so that the certain operations are performed. The processor 111 may control the optical portion 214 to obtain three-dimensional data of the object.

In an embodiment, the processor 111 may receive a control signal for the operation of the second camera from the data processing apparatus 120 and control turning on and off of the second camera accordingly.

In an embodiment, based on the scan mode of the scanner, the processor 111 may cause the second camera not to operate when the scanner operates in the second scan mode such that second image data is not obtained.

Hereinafter, the data processing apparatus 120 is described. The data processing apparatus 120 may also be referred to as an oral image processing apparatus.

The data processing apparatus 120 may include a processor 121, a memory 122, a user inputter 123, a communicator 124, a display 125, and an image processor 126.

The user inputter 123 may receive a user input for controlling the data processing apparatus 120. The user inputter 123 may include a user input device including a touch panel that detects a user's touch, a button that receives a user's push operation, or a mouse or a keyboard for specifying or selecting a point on a screen of the user inputter, and may also include a speech recognition apparatus for speech recognition or a motion sensor for motion recognition.

The communicator 124 may communicate with at least one external electronic apparatus via the wired or wireless communication network. The communicator 124 may communicate with the scanner 110 under control by the processor 121.

In detail, the communicator 124 may include at least one short-range communication module that performs communication according to communication standards, such as Bluetooth, Wi-Fi, BLE, NFC/RFID, Wi-Fi direct, UWB, or ZigBee. Also, the communicator 124 may further include a long-range communication module that communicates with a server for supporting long-range communication according to long-range communication standards.

Also, the communicator 124 may include at least one port for connection to an external electronic apparatus, for example, the scanner 110, with a wired cable.

In an embodiment, the communicator 124 may transmit a control signal to the scanner 110. The control signal transmitted to the scanner 110 may include at least one of a power-on command or a power-off command for the scanner 110, a command to cause the scanner 110 to enter the scan mode, or a command to turn off the operation of the second camera.

The display 125 may display a certain screen under the control by the processor 121. The display 125 may output a user interface screen for user input. The display 125 may display a screen including an oral image generated based on data obtained by scanning a patient's oral cavity or a plaster model of the oral cavity by using the scanner 110. Also, the display 125 may output a three-dimensional oral model generated from two-dimensional image data received from the scanner 110.

The image processor 126 may perform operations for generating and/or processing images. In detail, the image processor 126 may receive raw data obtained from the scanner 110 and generate a three-dimensional virtual model based on the received raw data.

The memory 122 may store at least one instruction. Also, the memory 122 may store at least one instruction executed by the processor. In addition, the memory may store at least one program executed by the processor 121. Also, the memory 122 may store data (e.g., raw data obtained through an oral scan) received from the scanner 110. Alternatively, the memory may store an oral image representing the oral cavity in three dimensions. According to an embodiment, the memory 122 may include one or more instructions for obtaining a three-dimensional oral model from the two-dimensional image data.

The processor 121 controls an intended operation to be performed by performing the at least one instruction stored in the memory 122. In this case, the at least one instruction may be stored in an internal memory included in the processor 121 or in the memory 122 included in the data processing apparatus separately from the processor 121.

In an embodiment, the processor 121 may execute the one or more instructions stored in the memory 122 to transmit a control signal to the scanner 110 and cause the scanner 110 to be controlled according to the control signal.

In an embodiment, the processor 121 may generate a three-dimensional oral model of the object by using first image data and second image data received from the scanner 110.

In an embodiment, the processor 121 may generate a three-dimensional oral model of the object by using the first image data and pattern light based on at least one of a user control signal, an identification signal indicating that the object is a metal, and the three-dimensional oral model of the object not being obtained from the first image data and the second image data.

In an embodiment, the processor 121 may generate a three-dimensional oral model of the object by obtaining depth information through finding a matching point between a pattern included in the first image data and a pattern of the pattern light.

In an embodiment, the processor 121 may interpret distortion of the second camera by using information that the second camera and the projector 401 being coaxial, and apply the distortion to pattern data of the projector 401. In an embodiment, the processor 121 may generate a three-dimensional oral model of the object by comparing the pattern data to which the distortion of the second camera is applied with the pattern included in the first image data.

In an embodiment, when image data obtained at the first radiance when the scanner 110 is in the first scan mode and image data obtained at the second radiance when the scanner 110 is in the second scan mode are received from the scanner 110 through the communicator 124, the processor 121 may generate a three-dimensional oral model of the object by using the first image data obtained by the first camera at the second radiance and the pattern data of the projector 401.

In an embodiment, in response to the projector 401 projecting NUV light by using the NUV light source, when the communicator 124 receives, from the scanner 110, image data of the NUV light reflected from the object, the image data being obtained by the first camera and the second camera, the processor 121 may generate a three-dimensional oral model, in which an area in a certain wavelength band is identified, from the image data of the NUV light reflected from the object.

According to an embodiment, when the processor 121 performs operations such as "extracting", "obtaining", and "generating", this may include not only the processor 121 directly performing the aforementioned operations by executing at least one instruction, but also controlling other components so that the aforementioned operations are performed.

In order to implement the embodiments of the present disclosure, the scanner 110 and the data processing apparatus 120 may include only some of the components shown in FIG. 11, or may include more components in addition to the components shown in FIG. 11.

Also, the data processing apparatus 120 may store and execute dedicated software linked to the scanner 110. In this case, the dedicated software may be referred to as a dedicated program, a dedicated tool, or a dedicated application. When the data processing apparatus 120 operates in conjunction with the scanner 110, the dedicated software stored in the data processing apparatus 120 may be connected to the scanner 110 and receive pieces of data obtained through an oral scan in real time.

Also, the dedicated software may transmit or receive a control signal to or from the scanner 110, and also perform at least one operation for obtaining, processing, storing, and/or transmitting an oral image. In this case, the dedicated software may be stored in the processor.

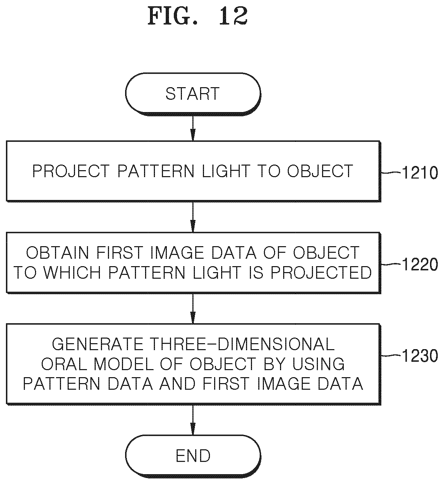

FIG. 12 is a flowchart of a data processing method according to an embodiment.

Referring to FIG. 12, in an embodiment, the scanner 110 may project pattern light to an object (operation 1210). In an embodiment, the scanner 110 may generate pattern light based on pattern data corresponding to a pattern to be generated by the projector 401 and project the pattern light to the object.

In an embodiment, the scanner 110 may obtain first image data of the object to which the pattern light is projected (operation 1220).

In an embodiment, the scanner 110 may include a first camera and a second camera spaced apart from each other, and the projector 401 may be positioned over or below one of the two cameras, for example, the second camera. The first camera and the second camera may obtain image data having different points of view with respect to the object to which the pattern light is projected.

In an embodiment, an optical path of the pattern light projected to the object may coincide in an opposite direction with an optical path of incident light to the second camera.

In an embodiment, the scanner 110 may transmit the pattern data and the first image data of the object to the data processing apparatus 120.

In an embodiment, the data processing apparatus 120 may generate a three-dimensional oral model of the object by using the pattern data and the first image data received from the scanner 110 (operation 1230).

In an embodiment, when the object is a metal, the data processing apparatus 120 may generate a three-dimensional oral model of the object by using the pattern data and the first image data of the object. In an embodiment, the data processing apparatus 120 may generate a three-dimensional oral model of the object by obtaining depth information through finding a matching point between a pattern included in the first image data and a pattern corresponding to the pattern data.

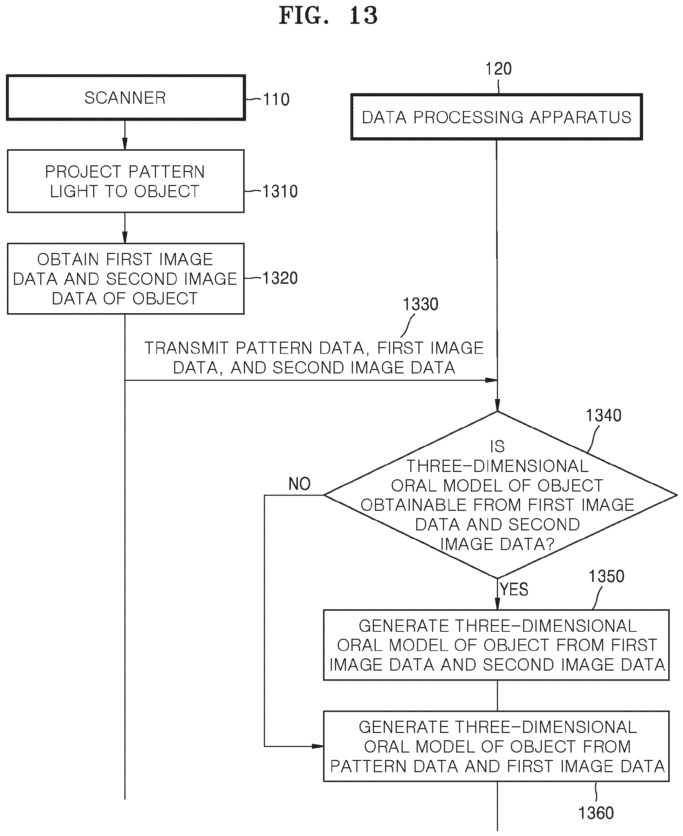

FIG. 13 is a flowchart of a data processing method according to an embodiment.

Referring to FIG. 13, in an embodiment, the scanner 110 may project pattern light to an object (operation 1310).

In an embodiment, the scanner 110 may obtain first image data and second image data of the object to which the pattern light is projected (operation 1320).

In an embodiment, the scanner 110 may transmit, to the data processing apparatus 120, at least one of pattern data and the second image data together with the first image data of the object. For example, in an embodiment, the scanner 110 may transmit, to the data processing apparatus 120, the pattern data and the first image data and the second image data of the object (operation 1330).

In an embodiment, the data processing apparatus 120 may identify whether a three-dimensional oral model of the object is obtainable by using the first image data and the second image data received from the scanner 110 (operation 1340).

In an embodiment, when the three-dimensional oral model of the object is obtainable by using the first image data and the second image data, the data processing apparatus 120 may generate the three-dimensional oral model of the object by using the first image data and the second image data (operation 1350).

In an embodiment, when the three-dimensional oral model of the object is not obtainable by using the first image data and the second image data, the data processing apparatus 120 may generate the three-dimensional oral model of the object by using the pattern data and the first image data (operation 1360).

The data processing method according to an embodiment of the present disclosure may be implemented in the form of program instructions executable through various computer means and recorded on a computer-readable medium. Also, an embodiment of the present disclosure may include a computer-readable storage medium having recorded thereon one or more programs including at least one instruction for executing the data processing method.

In addition, the aforementioned data processing method according to an embodiment of the present disclosure may be implemented as a computer program product including a computer-readable recording medium having recorded thereon a program for implementing the data processing method including emitting, by using a projector included in a scanner, pattern light generated based on pattern data, obtaining, by using a first camera included in the scanner, first image data of an object to which the pattern light is projected, and generating a three-dimensional oral model of the object by comparing a pattern included in the first image data with a pattern corresponding to the pattern data, wherein an optical path of the pattern light projected to the object coincides in an opposite direction with an optical path of incident light incident on a second camera included in the scanner.

The computer-readable storage medium may include a program command, a data file, a data structure, etc. alone or in combination. In this case, examples of the computer-readable storage medium may include a magnetic medium such as a hard disk, a floppy disk, and a magnetic tape, an optical medium such as a compact disk read-only memory (CD-ROM) and a digital video disk (DVD), a magneto-optical medium such as a floptical disk, and a hardware apparatus configured to store and execute program commands, such as ROM, random access memory (RAM), and flash memory.

In this case, a machine-readable storage medium may be provided in the form of a non-transitory storage medium. In this regard, the "non-transitory storage medium" may mean that the storage medium is a tangible apparatus. Also, the "non-transitory storage medium" may include a buffer in which data is temporarily stored.

According to an embodiment, the data processing method according to various embodiments provided in the present document may be provided by being included in a computer program product. The computer program product may be distributed in the form of a machine-readable storage medium (e.g., a CD-ROM). Alternatively, the computer program product may be distributed (e.g., downloaded or uploaded) through an application store (e.g., PlayStore^{™}), or directly or online between two user apparatuses (e.g., smartphones). In detail, the computer program product according to an embodiment of the disclosure may include a storage medium having recorded thereon a program including at least one instruction to perform the data processing method according to an embodiment of the disclosure.

While one or more embodiments have been described in detail, the scope of the present disclosure is not limited thereto, and it will be understood by those of ordinary skill in the art that various changes in form and details may be made therein without departing from the spirit and scope as defined by the following claims.

## Claims

1. A scanner comprising:
a communicator configured to transmit or receive information to or from a data processing apparatus;
a projector configured to emit pattern light generated based on pattern data; and
a data obtainer configured to obtain two-dimensional image data of an object,
wherein the data obtainer comprises a first camera and a second camera spaced apart from each other,
at least one of the first camera and the second camera is configured to obtain image data of the object to which the pattern light is projected, wherein the first camera is configured to obtain first image data, and the second camera is configured to obtain second image data,
an optical path of the pattern light projected to the object coincides in an opposite direction with an optical path of incident light incident on the second camera, and
the communicator is further configured to transmit, to the data processing apparatus, at least one of the second image data and the pattern data, together with the first image data.

2. The scanner of claim 1, wherein the scanner is configured to alternately operate in a first scan mode and a second scan mode during a scan operation, and
the data obtainer is further configured to,
when the scanner operates in the first scan mode, obtain two-dimensional image data at a first radiance, and
when the scanner operates in the second scan mode, obtain two-dimensional image data at a second radiance,
wherein the second radiance is less than the first radiance.

3. The scanner of claim 1, further comprising a processor,
wherein the processor is configured to, based on at least one of a control signal from the data processing apparatus and scan modes of the scanner, control turning on and/or off of the second camera.

4. The scanner of claim 4, wherein the projector comprises a near ultraviolet (NUV) light source,
at least one of the first camera and the second camera is a color camera comprising a color sensor or a black-and-white camera comprising a color filter,
in response to the projector projecting NUV light to the object by using the NUV light source, the first camera and the second camera are configured to obtain image data of the NUV light reflected from the object, and
the communicator is further configured to transmit, to the data processing apparatus, the image data of the NUV light reflected from the object.

5. A data processing apparatus comprising:
a communicator configured to transmit or receive information to or from a scanner comprising a projector, a first camera, and a second camera; and a processor configured to execute at least one instruction,
wherein the processor is further configured to
receive, from the scanner through the communicator, at least one of second image data obtained by the second camera and pattern data of the projector, together with first image data obtained by the first camera,
wherein an optical path of pattern light generated based on the pattern data and projected to the object coincides in an opposite direction with an optical path of incident light incident on the second camera, and
generate a three-dimensional oral model of the object by comparing a pattern included in the first image data with a pattern corresponding to the pattern data.

6. The data processing apparatus of claim 5, wherein the processor is further configured to generate the three-dimensional oral model of the object by obtaining depth information through finding a matching point between the pattern included in the first image data and the pattern corresponding to the pattern data.

7. The data processing apparatus of claim 5, wherein the processor is further configured to,
based on at least one of a user control signal, an identification signal indicating that the object is a metal, and the three-dimensional oral model of the object not being obtained from the first image data and the second image data, generate the three-dimensional oral model of the object by comparing the pattern included in the first image data with the pattern corresponding to the pattern data.

8. The data processing apparatus of claim 5, wherein the processor is further configured to receive, from the scanner through the communicator, image data obtained at a first radiance when the scanner is in a first scan mode, and image data obtained at a second radiance when the scanner is in a second scan mode, wherein the second radiance is less than the first radiance, and
generate the three-dimensional oral model by using, as the first image data, image data obtained by the first camera among the image data obtained at the second radiance.

9. The data processing apparatus of claim 5, wherein the processor is further configured to apply lens distortion of the second camera to the pattern data, and
generate the three-dimensional oral model of the object by comparing a pattern corresponding to the pattern data to which the lens distortion of the second camera is applied with the pattern included in the first image data.

10. The data processing apparatus of claim 5, wherein at least one of the first camera and the second camera is a color camera comprising a color sensor or a black-and-white camera comprising a color filter,
the communicator is further configured to, in response to the projector projecting near ultraviolet (NUV) light by using an NUV light source, receive, from the scanner, image data of the NUV light reflected from the object, the image data being obtained by the first camera and the second camera, and
the processor is further configured to generate, from the image data of the NUV light reflected from the object, the three-dimensional oral model in which an area in a certain wavelength band is identified.

11. A data processing method comprising:
emitting, by using a projector included in a scanner, pattern light generated based on pattern data;
obtaining, by using a first camera included in the scanner, first image data of an object to which the pattern light is projected; and
generating a three-dimensional oral model of the object by comparing a pattern included in the first image data with a pattern corresponding to the pattern data,
wherein an optical path of the pattern light projected to the object coincides in an opposite direction with an optical path of incident light incident on a second camera included in the scanner.

12. The data processing method of claim 11, wherein the generating of the three-dimensional oral model of the object comprises generating the three-dimensional oral model of the object by obtaining depth information through finding a matching point between the pattern included in the first image data with the pattern corresponding to the pattern data.

13. The data processing method of claim 11, wherein the generating of the three-dimensional oral model of the object comprises,
based on at least one of a user control signal, an identification signal indicating that the object is a metal, and the three-dimensional oral model of the object not being obtained from the first image data and the second image data, generating the three-dimensional oral model of the object by comparing the pattern included in the first image data with the pattern corresponding to the pattern data.

14. The data processing method of claim 11, wherein the obtaining of the first image data and second image data comprises:
when the scanner is in a first scan mode, obtaining image data at a first radiance; and
when the scanner is in a second scan mode, obtaining image data at a second radiance, wherein the second radiance is less than the first radiance, and
the generating of the three-dimensional oral model of the object comprises generating the three-dimensional oral model by using, as the first image data, image data obtained by the first camera among the image data obtained at the second radiance.

15. The data processing method of claim 11, further comprising applying lens distortion of the second camera to the pattern data,
wherein the generating of the three-dimensional oral model of the object comprises
generating the three-dimensional oral model of the object by comparing a pattern corresponding to the pattern data to which the lens distortion of the second camera is applied with the pattern included in the first image data.

16. The data processing method of claim 11, wherein one of the first camera and the second camera is a color camera comprising a color sensor or a black-and-white camera comprising a color filter, and
the data processing method further comprises:
projecting, by the projector, near ultraviolet (NUV) light by using an NUV light source;
obtaining, by using the first camera and the second camera, image data of the NUV light reflected from the object; and
generating, from the image data of the NUV light reflected from the object, the three-dimensional oral model in which an area in a certain wavelength band is identified.
